(19) **Europäisches Patentamt / European Patent Office / Office européen des brevets**

(11) **EP 4 340 720 B1**

(12) **FASCICULE DE BREVET EUROPEEN**

(45) Date de publication et mention
de la délivrance du brevet:
**05.11.2025 Bulletin 2025/45**

(21) Numéro de dépôt: **22724236.9**

(22) Date de dépôt: **11.05.2022**

(51) Classification Internationale des Brevets (IPC):
**A61B 5/11** (2006.01) **A61B 5/113** (2006.01)
**A61B 7/00** (2006.01) **A61B 7/04** (2006.01)

(52) Classification Coopérative des Brevets (CPC):
**A61B 5/1102**

(86) Numéro de dépôt international:
**PCT/FR2022/050904**

(87) Numéro de publication internationale:
**WO 2022/243625 (24.11.2022 Gazette 2022/47)**

(54) **DISPOSITIF POUR LA MESURE DE SIGNAUX VITAUX PERIODIQUES EMIS PAR UN INDIVIDU, ASSOCIE A UN EQUIPEMENT DE SECURITE D'UN VEHICULE**

VORRICHTUNG ZUR MESSUNG VON PERIODISCHEN VITALSIGNALEN, DIE VON EINER PERSON AUSGESENDET WERDEN, UND MIT EINER SICHERHEITSVORRICHTUNG EINES FAHRZEUGS AUSGESTATTET IST

DEVICE FOR MEASURING PERIODIC VITAL SIGNALS EMITTED BY AN INDIVIDUAL, ASSOCIATED WITH A SAFETY APPARATUS OF A VEHICLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.05.2021 FR 2105201**
**23.06.2021 FR 2106682**

(43) Date de publication de la demande:
**27.03.2024 Bulletin 2024/13**

(73) Titulaire: **Wormsensing**
**38040 Grenoble Cedex 9 (FR)**

(72) Inventeurs:
• **STEWART, Paul**
  **33110 Le Bouscat (FR)**
• **CADIEUX, Catherine**
  **38000 Grenoble (FR)**
• **LORNE, Thomas**
  **13510 Eguilles (FR)**

(74) Mandataire: **IP Trust**
**2, rue de Clichy**
**75009 Paris (FR)**

(56) Documents cités:
**EP-A1- 3 144 074** **WO-A1-2016/026028**
**CN-A- 106 500 826** **CN-A- 106 725 395**
**US-A1- 2013 033 382**

• **DELOBELLE P ET AL: "True Young modulus of Pb(Zr,Ti)O3 films measured by nanoindentation", APPLIED PHYSICS LETTERS, AMERICAN INSTITUTE OF PHYSICS, 2 HUNTINGTON QUADRANGLE, MELVILLE, NY 11747, vol. 85, no. 22, 1 January 2004 (2004-01-01), pages 5185 - 5187, XP012063583, ISSN: 0003-6951, DOI: 10.1063/1.1827331**
• **TSUCHIYA KAZUYOSHI ET AL: "Fabrication of Smart Material PZT Thin Films by RF Magnetron Sputtering Method in Micro Actuators", JSME INTERNATIONAL JOURNAL. SERIES A, SOLID MECHANICS AND MATERIAL ENGINEERING, 1 January 2006 (2006-01-01), Tokyo, pages 201 - 208, XP055871211, Retrieved from the Internet <URL:https://www.jstage.jst.go.jp/article/jsmea/ 49/2/49_2_201/_pdf/-char/en> [retrieved on 20211209], DOI: 10.1299/jsmea.49.201**

## Description

DOMAINE DE L'INVENTION

La présente invention concerne le domaine de la collecte de signaux vitaux périodiques émis par le corps humain, notamment les pulsations cardiaques ou le rythme respiratoire. Elle concerne en particulier un dispositif muni d'un capteur de vibration, lequel dispositif est associé à un équipement de sécurité (par exemple, la ceinture de sécurité) d'un véhicule et permet la mesure des pulsations cardiaques de l'utilisateur.

ARRIERE PLAN TECHNOLOGIQUE DE L'INVENTION

Les accidents de la route sont un problème majeur pour les pays développés. Parmi les principales causes d'accident se trouvent la fatigue et l'endormissement au volant. Au sein de l'Union Européenne (UE), on évalue que ces derniers seraient responsables de 20% à 35% des accidents sérieux et de près de 6000 morts par an. L'UE estime que l'intégration de détecteurs de fatigue à bord des véhicules permettrait de sauver 4000 vies et d'éviter des dizaines de milliers de blessés chaque année.

Parallèlement à cela, on observe une accélération de la recherche et du développement autour de la thématique du véhicule autonome. Si, dans les faits, la conduite autonome est encore loin d'être opérationnelle pour une mise sur le marché, tout semble indiquer qu'une première étape de « conduite autonome partielle » sous la responsabilité d'un conducteur deviendra courante dans les années à venir. Dans ce contexte, il sera donc nécessaire de s'assurer que le conducteur dispose de toutes ses capacités de vigilance et de réaction, si ce dernier devait reprendre la main dans une situation d'urgence. C'est dans cette optique que plusieurs organismes publics et privés, incluant les constructeurs et les équipementiers automobiles, tentent de trouver aujourd'hui des solutions viables de détection automatique de la fatigue dans les véhicules.

Certaines solutions envisagées, telles que les montres, bracelets ou ceintures vestimentaires connectés sont trop invasives pour l'utilisateur et n'assurent le suivi des variables physiologiques du conducteur que si ce dernier pense à les porter et/ou à les connecter.

D'autres solutions proposent d'intégrer un module de mesure de la fréquence cardiaque sur la ceinture de sécurité du véhicule, au niveau du torse du conducteur. Par exemple, le document CN106725395 propose un module de mesure de la fréquence cardiaque qui comprend deux électrodes métalliques prenant en sandwich la sangle en polyester de la ceinture de sécurité. Les pulsations cardiaques forcent un matériau isolant disposé entre les deux électrodes métalliques à se contracter : la distance entre les deux électrodes métalliques change, modifiant ainsi la valeur de capacitance et donnant une information sur la fréquence cardiaque du conducteur. Le document CN106500826A décrit un autre dispositif permettant d'enregistrer un signal vital périodique.

OBJET DE L'INVENTION

La présente invention vise également une solution associée à un équipement de sécurité d'un véhicule. Elle concerne en particulier un dispositif compact et sensible, muni d'un capteur de vibration, apte à capter et analyser les signaux vitaux périodiques d'un individu dans son véhicule.

BREVE DESCRIPTION DE L'INVENTION

L'invention concerne un dispositif selon la revendication 1.

Selon d'autres caractéristiques avantageuses et non limitatives de l'invention, prises seules ou selon toute combinaison techniquement réalisable :

- l'organe d'atténuation acoustique comprend un capot, composé d'un matériau souple présentant une dureté comprise entre 10 Shore 00 et 80 Shore 00, et solidaire de la couche support par sa périphérie ;
- le capot est hétérogène et comprend un deuxième matériau rigide choisi parmi les métaux ou les polymères présentant une dureté comprise entre 10 Shore D et 80 Shore D ;
- le dispositif comprend un organe d'atténuation mécanique, sur ou intégré en tout ou partie dans l'organe d'atténuation acoustique, ledit organe d'atténuation mécanique étant destiné à être en contact, direct ou indirect, avec l'équipement de sécurité ;
- l'organe d'atténuation mécanique comprend au moins un amortisseur et optionnellement un corps formant une masse ;
- la couche active de l'empilement de couches présente une épaisseur inférieure ou égale à 20 microns et un module d'Young supérieur ou égal à 60 GPa ;
- le dispositif comprend une couche d'adaptation d'impédance (40), présentant une impédance acoustique comprise entre 5.105 Pa*s/m et 3.106 Pa*s/m, et disposée sur une face de la couche support opposée à celle en contact avec la couche de connexion électrique ;
- le matériau piézoélectrique de la couche active est choisi parmi les céramiques sous forme monocristalline, poly-cristalline ou composite ;
- les électrodes de contact présentent une épaisseur cumulée inférieure à deux fois l'épaisseur de la couche active ;
- la couche support est autoportée et présente une épaisseur inférieure ou égale à 500 microns ;
- la couche d'adaptation d'impédance présente une épaisseur supérieure ou égale à 10 microns ;
- la couche de connexion électrique est formée par un interposeur ou par un film conductif anisotrope ;

- la couche support inclut une membrane disposée sur une face du circuit imprimé opposée à celle en contact avec la couche de connexion électrique ;
- l'empilement de couches et la couche support présentent respectivement une première superficie et une deuxième superficie, dans le plan principal, la première superficie étant inférieure ou égale à 30% de la deuxième superficie ;
- la couche support comprend une structure de rigidification, solidaire d'une zone périphérique de ladite couche support, l'organe d'atténuation acoustique étant lui-même solidaire de la structure de rigidification ;
- le circuit imprimé comprend un élément de connexion filaire, pour raccorder le capteur de vibration à un terminal électronique ;
- le capteur de vibration comprend un joint périphérique ;
- le dispositif comprend en outre un terminal électronique raccordé au capteur de vibration, pour analyser et interpréter le signal brut et extraire le signal vital périodique ou un paramètre de sortie représentatif dudit signal vital périodique ;
- le terminal électronique comprend un étage analogique de conditionnement du signal brut mesuré par le capteur de vibration, un étage de conversion analogique à digital du signal issu de l'étage de conditionnement, un étage de traitement du signal digital, pour la mise en forme du signal digital et le calcul d'un paramètre de sortie représentatif dudit signal vital ;
- le terminal électronique comprend un étage de communication avec un système externe.

L'invention concerne également un système de sécurité d'un véhicule comprenant :

- un équipement de sécurité, associé à un siège et solidaire d'un châssis du véhicule en au moins un point de contact direct ou indirect,
- un dispositif pour la mesure d'au moins un signal vital périodique d'un individu, tel que ci-dessus, fixé à l'équipement de sécurité par une attache glissante, et
- au moins un absorbeur d'énergie mécanique placé au niveau d'au moins un point de contact, de manière à isoler l'équipement de sécurité des vibrations mécaniques du châssis.

L'équipement de sécurité peut être directement relié au châssis par au moins trois points de contact, et un absorbeur d'énergie mécanique est alors intégré à au moins un des points de contact. L'équipement de sécurité peut être relié au siège, lequel est solidaire du châssis par au moins un point de contact, et un absorbeur d'énergie mécanique est alors intégré audit point de contact.

## BREVE DESCRIPTION DES FIGURES

D'autres caractéristiques et avantages de l'invention ressortiront de la description détaillée de l'invention qui va suivre en référence aux figures annexées :

- La figure 1 présente un système de sécurité comprenant un dispositif pour la mesure d'au moins un signal vital périodique d'un individu dans un véhicule, conformément à l'invention ;
- Les figures 2a et 2b présentent tout ou partie d'un dispositif conforme à l'invention, respectivement en coupe schématique et en perspective ;
- Les figures 3a et 3b présentent tout ou partie d'un dispositif conforme à l'invention, respectivement en coupe schématique et en perspective ;
- La figure 4 présente différentes formes, en vue de dessus, du capteur de vibration pour un dispositif conforme à l'invention ;
- La figure 5 présente différentes configurations de dispositifs pour la mesure d'un signal vital périodique, conformes à l'invention ;
- La figure 6a présente deux exemples d'organe d'atténuation acoustique (i)(ii) et deux exemples d'organe d'atténuation mécanique (iii) (iv), pour un dispositif conforme à l'invention ; la figure 6b présente un dispositif conforme à l'invention, associé à un équipement de sécurité d'un véhicule ;
- La figure 7a présente un spectrogramme A mesuré par un capteur de vibration (seul) tel qu'inclus dans le dispositif de l'invention et un spectrogramme B mesuré par un dispositif conforme à l'invention ; la figure 7b présente le spectrogramme B, un spectrogramme B' extrait du spectrogramme B, un spectrogramme B" après application d'un filtre en fréquences, et un signal vital B‴ sous forme d'onde capté et traité par un dispositif conforme à l'invention.

Les mêmes références sur les figures pourront être utilisées pour des éléments de même type. Certaines figures comportent des représentations schématiques qui, dans un objectif de lisibilité, ne sont pas à l'échelle : en particulier, les épaisseurs des couches selon l'axe z ne sont pas à l'échelle par rapport aux dimensions latérales selon les axes x et y ; et les épaisseurs relatives des couches entre elles ne sont pas nécessairement respectées.

Les différentes possibilités (variantes et modes de réalisation illustrés et/ou détaillés dans la description à suivre) doivent être comprises comme n'étant pas exclusives les unes des autres et peuvent se combiner entre elles.

## DESCRIPTION DETAILLEE DE L'INVENTION

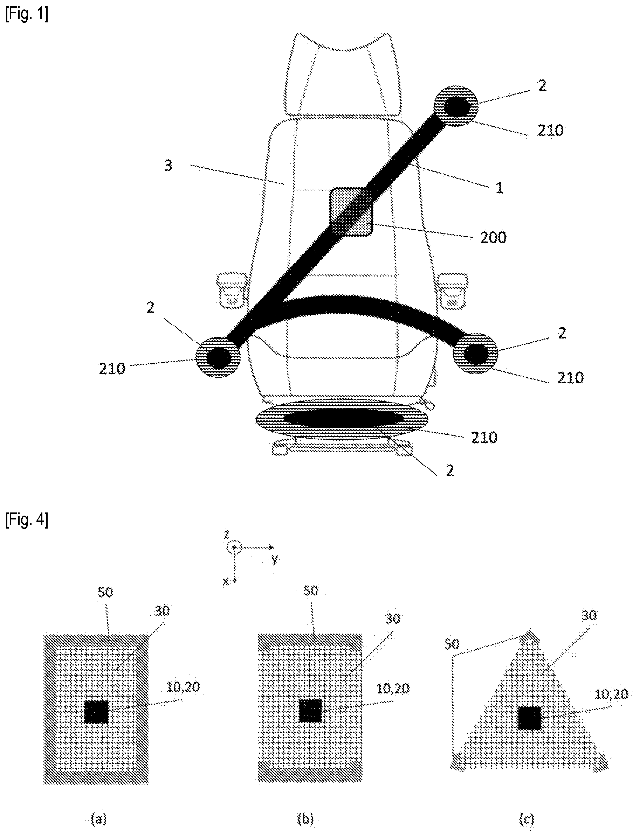

L'invention concerne un dispositif 200 pour la mesure d'au moins un signal vital périodique, régulier ou irrégulier, d'un individu. Le signal vital périodique peut notamment être le rythme cardiaque ou le rythme respiratoire. Le dispositif 200 est destiné à être fixé à un équipement de sécurité 1, dans un véhicule, de sorte que ledit dispositif 200 soit disposé entre l'individu et l'équipement 1 (figure 1). Par équipement de sécurité 1, on entend tout équipement destiné à sécuriser l'utilisateur sur un siège du véhicule, notamment une ceinture de sécurité, une ou des barre(s) de sécurité, un harnais de sécurité, etc. Le véhicule peut également s'entendre au sens large, et inclut tout mode de transport de personnes, roulant, volant, glissant ou flottant.

Le dispositif 200 est préférentiellement fixé à l'équipement de sécurité 1 par une attache glissante, c'est-à-dire une attache apte à se clipper sur l'équipement 1 pour immobiliser le dispositif 200 dans une position donnée, et apte à coulisser (lorsqu'elle est déclippée), pour permettre à chaque utilisateur d'ajuster la position du dispositif 200 sur son thorax, selon sa taille et sa corpulence. Eventuellement, le système d'attache pourra autoriser une latitude de déplacement autour de la position de fonctionnement, et ce pour le confort de l'utilisateur.

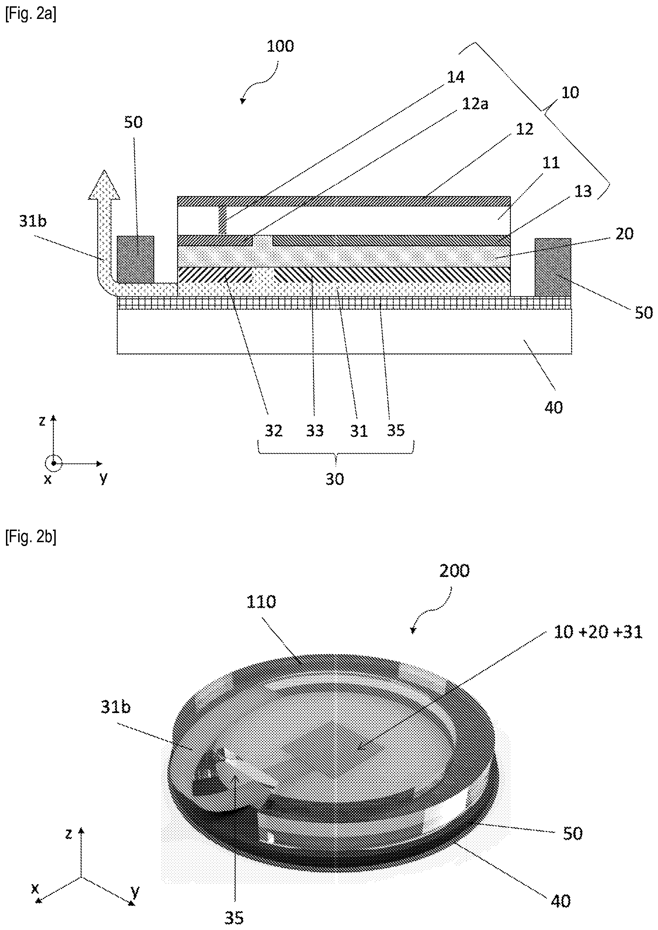

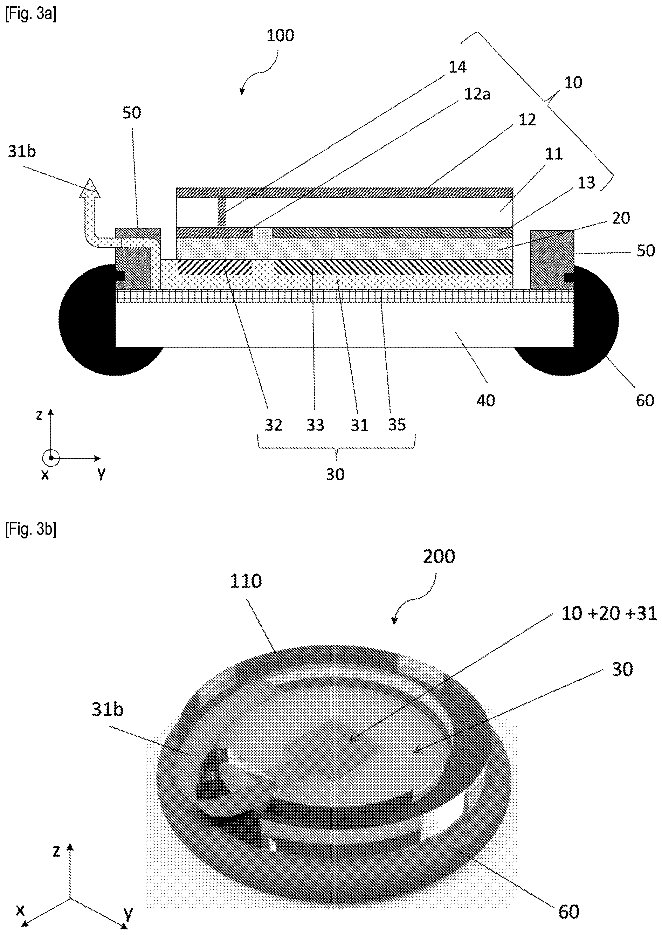

Le dispositif 200 comprend un capteur de vibration 100 et un organe d'atténuation acoustique 110. Différentes configurations de capteurs de vibration 100 conformes à la présente invention sont illustrées sur les figures 2a, 2b, 3a et 3b et vont maintenant être décrites.

Le capteur de vibration 100 comprend un empilement de couches 10 s'étendant parallèlement à un plan principal (x,y), c'est-à-dire que les faces principales de cet empilement 10 sont sensiblement parallèles au plan principal (x,y) et que l'épaisseur de l'empilement 10 se mesure selon un axe z normal audit plan principal. La dénomination de couche, dans la présente invention, sous-entend que l'épaisseur de la couche (ou de l'empilement de couches) est, en général, significativement inférieure aux dimensions latérales (dans le plan principal) de ladite couche.

L'empilement de couches 10 inclut une couche active 11 en matériau piézoélectrique, préférentiellement choisi parmi les céramiques piézoélectriques, sous une forme monocristalline, poly-cristalline ou composite (correspondant à une dispersion de poudre céramique piézoélectrique dans une matrice, polymère généralement). A titre d'exemple, on peut citer les céramiques suivantes : niobate de lithium ($LiNbO_3$), tantalate de lithium ($LiTaO_3$), niobate de potassium ($KNbO_3$), ($BaTiO_3$), quartz ($SiO_2$), niobate de magnésium-plomb titanate de plomb (PMN-PT), titano-zirconate de plomb (PZT), matériaux à base de potassium sodium niobium lithium antimoine (KNN-LS) ou modifiés par du titanate de calcium (KNN-LS-CT), matériaux à base de potassium sodium lithium niobium tantale antimoine (KNLNTS), titanate de bismuth sodium (BNKLBT), etc.

Comme cela est connu en soi, la couche active 11 en matériau piézoélectrique va se polariser (et donc générer une circulation de charges conduisant à un signal électrique mesurable) si elle subit une déformation, en particulier ici, déformation provoquée par la pulsation du signal vital périodique.

La couche active 11 présente avantageusement une épaisseur inférieure ou égale à 20 microns et un module d'Young supérieur ou égal à 60 GPa. Ces caractéristiques physiques confèrent à la couche active 11 un haut niveau de sensibilité (liée à sa faible épaisseur et au fait que la tension mesurée est d'autant plus grande, pour une déformation donnée, que le module d'Young est élevé) et au capteur 100 un fort ratio signal sur bruit, pour la détection d'ondes acoustiques dans les fréquences relatives aux signaux vitaux périodiques visés. La faible épaisseur de la couche active 11 favorise également la compacité du capteur 100.

L'épaisseur de la couche active 11 peut être inférieure ou égale à 10 microns, voire même inférieure ou égale à 5 microns, pour améliorer encore la sensibilité de détection des ondes acoustiques. On s'assurera de conserver une épaisseur de couche active 11 suffisante pour générer des tensions de polarisation typiquement supérieures à 500 microvolts lors d'une déformation.

Les dimensions latérales (dans le plan principal (x,y)) de la couche active 11 peuvent par exemple être choisies entre 500 microns et 50 mm, des faibles dimensions étant bien sûr privilégiées pour des questions de compacité du capteur de vibration 100.

L'empilement de couches 10 inclut également deux électrodes de contact 12,13, disposées sur une des faces de la couche active 11 ou sur les deux faces (à savoir, de part et d'autre de la couche active 11), pour permettre la libre circulation des charges, mises en mouvement par la polarisation (représentative du signal vital périodique) de ladite couche 11.

Préférentiellement, les électrodes de contact 12,13 présentent une épaisseur cumulée inférieure à deux fois l'épaisseur de la couche active 11, voire inférieure à l'épaisseur de la couche active 11 ; chaque électrode 12,13 présente donc avantageusement une épaisseur inférieure à 10 microns, voire inférieure à 5 microns.

Les électrodes de contact 12,13 pourront être formées à partir de matériaux métalliques purs (par exemple, Ag, Au, Pd, Pt, Cu, Ni, W ou Ti), d'alliages conducteurs ou de matériaux conducteurs 2D (par exemple, le graphène). Une barrière de diffusion (par exemple, en TiN, WN ou TaN) et une couche d'accroche (par exemple, en Cr ou Ti) pourront être prévues entre le matériau conducteur de chaque électrode 12,13 et la couche active 11.

Avantageusement, l'empilement de couches 10 est constitué de la couche active 11 et des deux électrodes de contact 12,13 uniquement.

Le capteur de vibration 100 comprend également une couche support 30, flexible, s'étendant parallèlement au plan principal (x,y) et incluant un circuit imprimé 31 comportant deux bornes électriques 32,33. Une couche de connexion électrique 20 (qui fait également partie du capteur de vibration 100) est disposée entre l'empilement de couches 10 et la couche support 30, pour connecter chaque électrode de contact 12,13 à une borne électrique 32,33.

La couche de connexion électrique 20 est avantageusement formée par un interposeur ou par un film conductif anisotrope (ACF). Dans tous les cas, l'objectif est que les deux électrodes de contact 12,13 de l'empilement de couches 10 puissent être atteintes au niveau d'une seule et même face de l'empilement 10 ; cette face (dite face inférieure) étant ensuite associée à la couche de connexion 20. Dans le cas où les électrodes de contact 12,13 sont respectivement disposées sur la face inférieure et l'autre face (dite face supérieure) de la couche active 11, il est avantageux de prévoir un via conducteur 14 traversant ladite couche active 11 et reliant électriquement l'électrode 12, disposée sur la face supérieure, à un plot 12a disposé sur la face inférieure et isolé électriquement de l'autre électrode 13 également disposée sur la face inférieure.

Un interposeur peut être composé de résine thermoplastique (isolant) et d'un matériau conducteur électrique (par exemple, du nickel) permettant le raccordement entre chaque électrode de contact 12,13 et une borne électrique 32,33.

Un film conductif anisotrope est classiquement composé de billes conductrices dispersées dans une matrice polymère isolante ; lorsqu'une pression ou une thermocompression est appliquée à l'ensemble empilement de couches 10 / ACF 20 / couche support 30, une conduction électrique verticale s'établit entre électrodes 12a,13 et bornes 32,33 (habituellement en surépaisseur) via les billes conductrices, alors que les zones intercalaires restent isolantes.

Il existe également des adhésifs conductifs anisotropes (ACA) qui pourraient être utilisés pour former la couche de connexion électrique 20. Ces adhésifs sont basés sur le même principe que le film conductif anisotrope (ACF) précité, à l'exception du fait que la matrice polymère est remplacée par un précurseur liquide capable d'être thermiquement activé pour former le polymère final (par polymérisation) ; le résultat final reste similaire à l'ACF (billes conductrices dispersées dans une matrice isolante), mais compte tenu du fait que l'application se fait en phase liquide, il est possible de réduire drastiquement l'épaisseur de la couche de connexion électrique 20.

Une solution plus basique peut également être envisagée : à savoir la mise en œuvre d'une pate conductrice pour connecter chaque électrode et plot de la face inférieure, à une borne 32,33 associée, et d'un matériau de remplissage isolant pour isoler électriquement les électrodes 12a,13 entre elles et les bornes 32,33 entre elles.

La couche de connexion électrique 20 est uniquement en contact avec l'une des faces principales de l'empilement de couches 10 ; les bords et l'autre face principale de l'empilement de couches 10 sont totalement libres, sans contact mécanique avec la couche de connexion 20.

La couche de connexion électrique 20 est donc au moins en partie composée d'un matériau conducteur électrique et procure une connexion verticale directe entre électrodes et bornes, à l'inverse d'une connexion par exemple par câbles ou fils éventuellement enrobés dans un isolant. L'absence de câbles améliore la sensibilité du capteur de vibration 100, en évitant l'introduction de raideur supplémentaire dans la structure, liée aux câbles et aux soudures associées.

Préférentiellement, la couche de connexion électrique 20 est donc en contact direct et homogène contre l'intégralité d'une face principale de l'empilement de couches 10. Du côté de son autre face, la couche 20 est avantageusement en contact direct et homogène contre une face de la couche support 30.

La couche de connexion électrique 20 présente typiquement une épaisseur inférieure à 50 microns, en particulier une épaisseur comprise entre 1 micron et 10 microns.

La couche support 30 est une couche autoportée, qui présente avantageusement une épaisseur inférieure ou égale à 500 microns. Cela lui confère la flexibilité requise.

Selon une variante, la couche support 30 est essentiellement composée par le matériau formant le circuit imprimé 31 : par exemple, un composite de résine époxyde renforcé de fibres de verre.

Selon une autre variante, la couche support 30 comprend également une membrane 35, le circuit imprimé 31 étant alors situé entre la membrane 35 et la couche de connexion électrique 20 (figures 2a et 3a). Le matériau de la membrane 35, et son épaisseur, peuvent ainsi être choisis et ajustés de manière à conférer la flexibilité visée à la couche support 30. La membrane 35 peut par exemple être formée en métal, en chlorure de polyvinyle (PVC), ou en époxy et fibres de verres. A titre d'exemple, la membrane 35 (lorsqu'elle est présente) peut avoir une épaisseur comprise entre 50 et 300 microns, et le circuit imprimé 31 peut avoir une épaisseur comprise entre 30 et 200 microns.

Typiquement, la couche support 30 présente une raideur comprise entre 1150000 N/m et 6900000 N/m. Le caractère flexible de la couche support 30, lié à son épaisseur et à sa raideur, permet de transmettre efficacement une déformation à la couche active 11, à chaque pulsation du signal vital.

De manière avantageuse, l'empilement de couches 10 et la couche support 30 présentent respectivement une première superficie et une deuxième superficie, dans le plan principal (x,y), la première superficie étant inférieure ou égale à 30% de la deuxième superficie. L'empilement de couches 10 peut être disposé en partie centrale de la couche support 30, notamment pour une facilité d'assemblage, ou en périphérie pour interférer le moins possible avec la déformation de ladite couche support 30, laquelle déformation est générée par la pulsation périodique du signal vital que l'on cherche à mesurer ; l'objectif global est d'optimiser la déformation subie par l'empilement de couches 10, cela en fonction de la géométrie du capteur de vibration 100. Notons que, bien qu'illustré sous une forme carrée, l'empilement de couches 10 du capteur de vibration 100, peut, bien évidemment, présenter une forme quelconque.

Selon un premier mode de mise en œuvre du capteur de vibration 100, la couche support 30 est destinée à être en contact avec l'individu : la couche support 30 va alors se déformer du fait de la pulsation périodique du signal vital, et transmettre cette déformation à la couche active 11 de l'empilement 10.

Selon un deuxième mode de mise en œuvre, le capteur de vibration 100 comprend en outre une couche d'adaptation d'impédance 40, qui présente une impédance acoustique idéalement comprise entre $5.10^5$ Pa*s/m et $3.10^6$ Pa*s/m. Cette impédance acoustique est sciemment choisie proche de l'impédance acoustique des muscles et de la graisse (impédance comprise entre $1,3.10^6$ et $1,5.10^6$ Pa*s/m), de manière à favoriser la transmission des pulsations du signal vital à la couche support 30. Par exemple, la couche d'adaptation d'impédance 40 peut être formée en silicone (impédance acoustique $1,6.10^6$ Pa*s/m) ou en bioplastique, par exemple de marque Ecoflex® (impédance acoustique $1,053.10^6$ Pa*s/m).

La couche d'adaptation d'impédance 40 est disposée contre la couche support 30, sur une face de ladite couche support 30 opposée à celle en contact avec la couche de connexion électrique 20. La couche d'adaptation d'impédance 40 présente typiquement une épaisseur supérieure ou égale à 10 microns, par exemple comprise entre 50 microns et 5 mm. Lorsque la couche support 30 comprend une membrane 35, cette dernière est en contact avec la couche d'adaptation d'impédance 40.

La couche d'adaptation d'impédance 40 est destinée à être en contact avec l'individu. En plus de transmettre efficacement les pulsations du fait de son appariement d'impédance avec les tissus corporels, cette couche 40 favorise également le maintien du capteur 100 contre l'individu car son matériau souple et déformable a tendance à « adhérer » à la surface de contact, par frottement d'adhérence sur les vêtements. La présence de la couche d'adaptation d'impédance 40, dans le deuxième mode de mise en œuvre du capteur 100, est donc particulièrement favorable lorsque l'environnement de mesure est bruyant autour de l'individu dont il faut capter le signal vital.

Dans l'un ou l'autre des modes de mise en œuvre décrits, il peut être avantageux que le capteur de vibration 100 comprenne un joint périphérique 60 entourant au moins la couche d'adaptation d'impédance 40 (lorsqu'elle est présente), comme illustré sur les figures 3a et 3b, ou entourant tout ou partie de la couche support 30 (en l'absence de couche d'adaptation d'impédance 40). Ce joint 60 permet d'accommoder la topologie locale lorsque le capteur 100 est placé au contact de l'individu.

La couche support 30 du capteur de vibration 100 peut également comprendre une structure de rigidification 50, solidaire d'une zone périphérique de la couche support 30. La structure de rigidification 50 a pour fonction d'immobiliser la périphérie de la couche support 30 et de la couche d'adaptation d'impédance 40 (si elle est présente), et ainsi d'accentuer leur déformation générée par la pulsation périodique du signal vital que l'on cherche à mesurer. La structure de rigidification 50 peut prendre diverses formes telles que par exemple :

- un cadre continu (figure 4(a)), avantageusement un anneau (comme illustré sur la figure 2b), mais éventuellement un rectangle, un triangle ou autre polygone ; ou
- un cadre discontinu, composé de deux zones rigides (figure 4(b)), de trois zones rigides (figure 4(c)), voire plus.

La structure de rigidification est avantageusement formée dans un matériau présentant une dureté supérieure à 30 Shore D, tel que le PET (polytéréphtalate d'éthylène), PMMA (polyméthacrylate de méthyle), PU (polyuréthane), PVC (polychlorure de vinyle), PP (polypropylène), etc.

Compte tenu de l'épaisseur totale réduite de l'ensemble comprenant l'empilement de couches 10, la couche de connexion 20, la couche support 30 et potentiellement la couche d'adaptation d'impédance 40, il peut être judicieux de prévoir un système facilitant la manipulation du capteur 100 et favorisant sa robustesse : la structure de rigidification 50 participe à un tel système.

Le dispositif 200 selon l'invention comprend, outre le capteur de vibration 100 qui vient d'être décrit, un organe d'atténuation acoustique 110, destiné à être placé entre l'équipement de sécurité 1 et le capteur de vibration 100.

Cet organe 110 est disposé au-dessus et à distance de l'empilement de couches 10 du capteur de vibration 100, et il est solidaire de la couche support 30. Parce qu'il est situé à distance (selon l'axe z sur les figures) de l'empilement de couches 10 (sans contact avec l'empilement 10, donc), typiquement à une distance de l'ordre de 0,1mm à 10mm, il ne perturbe pas la déformation de celui-ci en liaison avec la couche support 30.

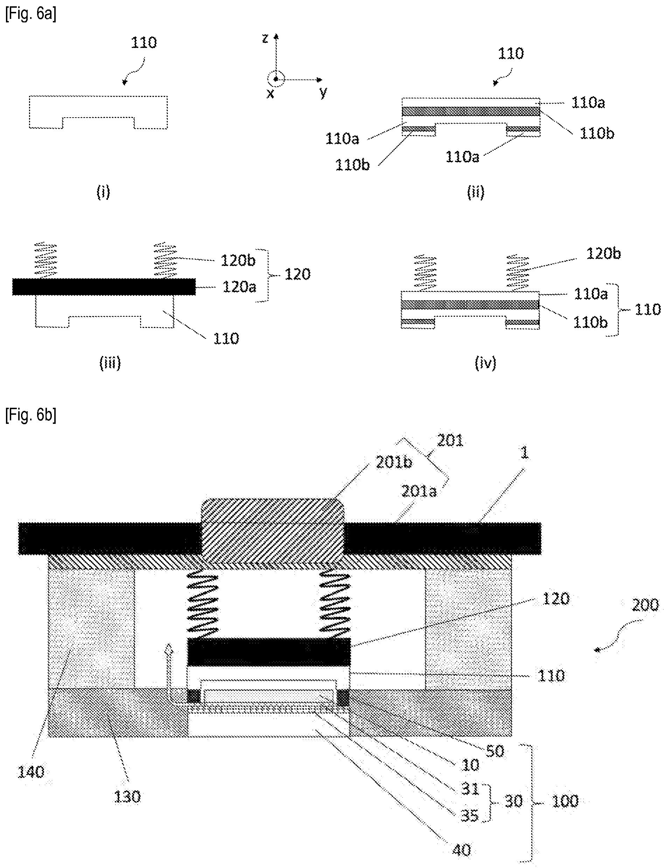

L'organe d'atténuation acoustique 110 prend avantageusement la forme d'un capot (figure 6a (i),(ii)), dont la périphérie est fixée à la couche support 30, ou lorsqu'elle est présente, à la structure de rigidification 50. A titre d'exemple, l'épaisseur du capot, au-dessus de l'empilement de couche 10, peut varier entre 0.1mm et 20mm.

L'organe d'atténuation acoustique 110 a pour objectif d'isoler le capteur acoustique 100 (et plus particulièrement la couche support 30 qui se déforme avec les vibrations et la couche active 11 sensible auxdites déformations) des perturbations acoustiques environnantes, qui se propagent dans l'air : à savoir, le son du moteur, le son de la route, les frottements de l'air sur la carrosserie, les voix des passagers dans le véhicule, la radio, etc. Il est préférentiellement composé d'un matériau souple de type élastomère tel que le silicone, le sorbothane ou le caoutchouc. Plus généralement, le matériau souple de l'organe d'atténuation acoustique 110 peut être qualifié par sa dureté Shore : il présente une dureté comprise entre 10 Shore 00 et 80 Shore 00. En plus de sa fonction d'atténuation acoustique, l'organe 110 participe à la robustesse du dispositif 200 en protégeant notamment la couche active 11 du capteur de vibration 100.

Selon une variante, l'organe d'atténuation acoustique 110 peut comprendre plusieurs types de matériaux. S'il se présente sous la forme d'un capot, on le qualifie alors de capot hétérogène. Le deuxième matériau est choisi rigide, de nature métallique ou polymère (par exemple, l'aluminium, le PVC). Si le deuxième matériau est un polymère, on choisira sa dureté préférentiellement entre 10 Shore D et 80 Shore D.

Le capot hétérogène 110 est formé d'une alternance d'au moins une première couche 110a en matériau souple et d'au moins une deuxième couche 110b en matériau rigide comme illustré sur la figure 6a (ii). Le capot hétérogène peut également être composé d'un ou plusieurs matériau(x) poreux, comme par exemple une mousse de polyuréthane.

Avantageusement, le dispositif 200 comprend en outre un organe d'atténuation mécanique 120 dont le rôle est d'isoler le capteur de vibration 100, des vibrations mécaniques générées par le moteur du véhicule, par les conditions de route et/ou par les mouvements de l'utilisateur, et transmises à l'équipement de sécurité 1 par l'intermédiaire du châssis. L'organe d'atténuation mécanique 120 est donc destiné à être en contact (direct ou indirect) avec l'équipement de sécurité 1. Cet organe d'atténuation mécanique 120 peut être disposé sur l'organe d'atténuation acoustique 110 ou intégré en tout ou partie dans ce dernier.

Selon une première option, l'organe d'atténuation mécanique 120 est composé d'un corps 120a formant une masse et d'au moins un amortisseur 120b (figure 6a (iii)). Le corps 120a est disposé contre l'organe d'atténuation acoustique 110 et le (ou les) amortisseur(s) est(sont) placé(s) du côté de l'équipement de sécurité 1.

L'amortisseur 120b est défini par une raideur k comprise entre 0 (frottement seul) et 7 N/mm, et par un coefficient de frottement f compris entre 0 (raideur seule) et 0.6. Chaque amortisseur 120b peut par exemple être formé par un ressort métallique, un pilier en résine, caoutchouc ou silicone, ou encore un élément amortisseur simple, mixte (caoutchouc / métal) ou hydraulique.

Le corps 120a présente une masse m comprise entre 1g et 1kg. L'organe d'atténuation mécanique 120 forme un système « masse - ressort - piston » agissant comme un filtre mécanique passe-haut. En ajustant la masse m, la raideur k et le coefficient de frottement f, on peut changer les propriétés du filtre mécanique et atténuer spécifiquement les vibrations mécaniques transmises à l'équipement de sécurité 1.

Notons que la masse de l'organe d'atténuation acoustique 110 et celle du capteur de vibration 100 doivent être prises en considération, et additionnées à la masse du corps 120a pour aboutir aux propriétés du filtre mécanique souhaitées.

Il est visé que le filtre mécanique coupe/atténue les fréquences parasites situées dans la zone d'intérêt. Ainsi, dans le cas idéal, on veut que la fréquence de coupure ($F_C \propto \sqrt{\frac{k}{m}}$) du filtre se situe aux alentours des 150Hz pour couper toutes les fréquences parasites provenant du châssis (vibrations mécaniques), et que son taux d'amortissement ($\tau \propto \frac{f}{\sqrt{km}}$) soit le plus proche de 1 pour avoir la meilleure atténuation possible. Dans la pratique, il y a bien sûr des compromis à faire entre ce cas idéal et les contraintes de design du dispositif 200.

Selon une deuxième option, l'organe d'atténuation mécanique 120 est partiellement intégré dans l'organe d'atténuation acoustique 110 c'est-à-dire que le corps 120a consiste en une couche en matériau rigide 110b qui compose ledit organe d'atténuation acoustique 110 (par exemple, sous forme d'un capot hétérogène, comme illustré sur la figure 6a (iv)). La partie amortisseuse 120b de l'organe d'atténuation mécanique 120 est alors fixée sur l'organe d'atténuation acoustique 110 et peut être formée par les différents éléments énoncés dans la première option.

Selon une troisième option, l'organe d'atténuation mécanique 120 est totalement intégré dans l'organe d'atténuation acoustique 110. Pour cela, l'organe d'atténuation mécanique 120 (inclus dans l'organe d'atténuation acoustique 110) peut être formé en matériaux composites présentant des propriétés viscoélastiques.

Le dispositif 200 selon l'invention peut présenter une forme générale circulaire, carrée, rectangulaire ou polygonale, dans le plan principal (x,y). Comme illustré sur la figure 6b, il est destiné à être disposé entre l'équipement de sécurité 1 et l'individu assis dans le véhicule. La face du dispositif 200, située du côté de la couche support 30 du capteur de vibration 100 (et du côté de la couche d'adaptation d'impédance 40 lorsque cette dernière est présente), est placée contre le thorax de l'individu, préférentiellement dans une zone où les pulsations cardiaques ou le rythme respiratoire sont palpables. L'autre face du dispositif 200, situé du côté de l'organe d'atténuation acoustique 110 (et de l'organe d'atténuation mécanique 120, s'il est présent), est maintenue contre l'équipement de sécurité 1. Le contact entre le dispositif 200 et l'équipement 1 est préférentiellement effectué par le biais d'une attache glissante 201 (figure 6b) : en particulier, la face du dispositif 200 est solidaire (collée ou fixée mécaniquement) à un élément support 201a de l'attache 201, lequel élément est fixé à l'équipement de sécurité 1 par un clip glissant 201b.

Le dispositif 200 selon l'invention présente l'avantage d'atténuer fortement les fréquences situées en-dehors de la gamme de fréquences à mesurer (gamme de fréquences typiquement entre 0,2 Hz et 500 Hz pour les rythmes cardiaque et respiratoire, voire fréquences inférieures ou égales à 70 Hz) et également d'atténuer des fréquences parasites situées dans la gamme de fréquences d'intérêt. Il a en particulier été observé que la parole et d'autres sons d'environnement ne viennent pas polluer le signal mesuré. L'environnement sonore de l'individu au moment de la prise de mesure n'a donc pas besoin d'être calme et silencieux. Cela est possible grâce à la structure particulière du capteur de vibration 100 ainsi que du fait de la présence de l'organe d'atténuation acoustique 110.

De plus, la présence de l'organe d'atténuation mécanique 120 (ou comme cela sera décrit plus loin en référence au système de sécurité, objet de la présente invention, la présence d'au moins un absorbeur d'énergie mécanique 210) atténue significativement les vibrations mécaniques produites par le moteur en fonctionnement et éventuellement les irrégularités de la route, vibrations qui sont transmises à l'équipement de sécurité 1 via le châssis du véhicule. La neutralisation de ces vibrations mécaniques parasites autorise une capture fiable et reproductible des signaux vitaux de l'individu par le capteur de vibration 100.

Avantageusement, le dispositif 200 est associé à un tissu 130 et une mousse 140 pour améliorer le confort de l'utilisateur (figure 6b). Le tissu 130 peut par exemple border la couche support 30 et la couche d'adaptation d'impédance 40 si elle est présente ; il peut, de manière générale, border tout ou partie du capteur de vibration 100 et ainsi procurer une surface lisse et uniforme de contact avec l'individu, qui va permettre d'accommoder les morphologies d'utilisateur, les types de vêtements et/ou les variations d'ajustement des équipements de sécurité 1. La mousse 140 fait typiquement le lien entre le tissu 130 et l'attache 201 ; elle est souple et déformable et ne modifie pas ou très peu le filtre mécanique défini par l'organe d'atténuation mécanique 120.

Le tissu 130 pourra être formé de coton, de nylon, ou encore de polyéthylène ; la mousse 140 pourra être formée de polyuréthane, de polyéthylène ou de polystyrène.

Le dispositif 200, associé à un équipement de sécurité 1 dans un véhicule, permet la mesure d'au moins un signal brut représentatif d'un signal vital périodique de l'individu installé dans ledit véhicule.

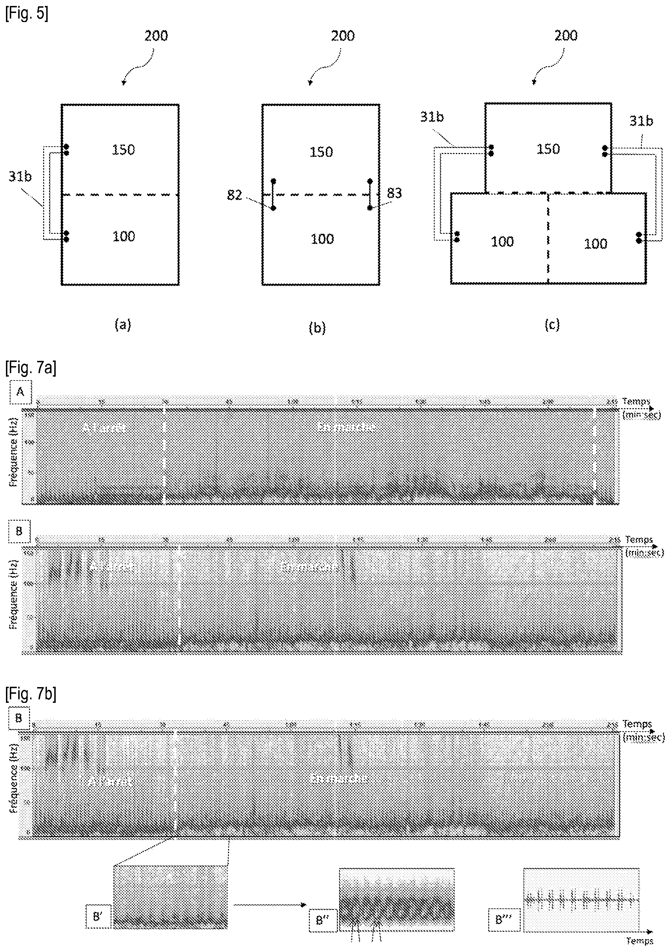

Pour analyser et interpréter le signal brut puis extraire le signal vital périodique ou des informations relatives à ce signal vital, le dispositif 200 comprend en outre un terminal électronique 150 raccordé électriquement au capteur de vibration 100. Notons que le dispositif 200 peut comporter un capteur de vibration 100 (figure 5 (a), (b)) ou une pluralité (deux, voire plus) de capteurs 100 raccordés au terminal électronique 150 (figure 5 (c)). Lorsqu'il y a plusieurs capteurs 100, il est possible de mesurer un même signal ou des signaux vitaux différents (rythme cardiaque et respiration) de l'individu.

Pour connecter le capteur de vibration 100 et le terminal électronique 150, le circuit imprimé 31 du capteur de vibration 100 peut comprendre un élément de connexion filaire 31b, par exemple un cordon sous forme de nappe, comme illustré sur les figures 2a, 2b, 3a, 3b et 5(a). L'embout de l'élément de connexion filaire 31b comporte des prises de contact électrique, reliées aux bornes électriques 32,33 du circuit imprimé 31, et qui peuvent être connectées au terminal électronique 150.

Le terminal électronique 150 peut être accolé au capteur 100 ou situé à distance du capteur 100, notamment sur un module de fixation à l'équipement de sécurité 1 ou à une autre partie du véhicule. Le terminal électronique 150 peut être connecté ou intégré à un système externe plus complexe, tel qu'un moniteur, fixe ou éventuellement transportable.

Alternativement, le terminal électronique 150 peut être disposé sur l'organe d'atténuation acoustique 110 et former tout ou partie du corps 120a de l'organe d'atténuation mécanique 120. Cette configuration assure une grande compacité du dispositif 200. Dans ce cas, on peut envisager non plus un élément de connexion filaire 31b pour relier électriquement le capteur de vibration 100 et le terminal 150, mais des prises de contact 82,83 remontant verticalement depuis le circuit imprimé 31 du capteur 100 jusqu'à la surface de l'organe d'atténuation acoustique 110, via la structure de rigidification 50 par exemple (figure 5(b)).

Le terminal 150 peut comprendre différents étages électroniques lui permettant d'analyser et d'interpréter le signal brut mesuré par le capteur de vibration 100. Un étage analogique de conditionnement du signal brut mesuré par le capteur de vibration 100 va tout d'abord amplifier et filtrer le signal électrique reçu du capteur 100. Cet étage est typiquement composé d'un premier bloc de type amplification de charge dont le rapport des résistances fixe le gain d'amplification du signal électrique reçu du capteur 100, et d'un deuxième bloc de type filtre Sallen & Key permettant de filtrer les fréquences au-delà du spectre acoustique des signaux vitaux visés. Le terminal électronique 150 comprend ensuite un étage de conversion analogique à digital, du signal issu de l'étage de conditionnement. Puis, un étage de traitement du signal digital, composé d'un microcontrôleur, réalise la mise en forme du signal par le calcul d'une fonction enveloppe de type énergie de Shanon. Enfin, à partir du signal mis en forme, le paramètre de sortie d'intérêt, représentatif dudit signal vital, peut être calculé.

Les données collectées, relatives au signal vital ou au paramètre de sortie d'intérêt, peuvent être interprétées en temps réel et déclencher la réponse d'un système secondaire compris dans le dispositif 200 ou externe. La réponse peut être un retour d'information (visuelle, acoustique, mécanique, vibratoire, etc) et/ou le déclenchement d'une ou plusieurs actions, par exemple :

- mécanique(s) : ouverture / fermeture d'un système,
- électrique(s) : allumage / extinction / variation d'un système, hydraulique, pneumatique, thermique, etc.

Dans tous les cas, la réponse du système secondaire vise à informer l'individu (typiquement le conducteur du véhicule), voire à l'alerter, si le signal vital détecté révèle qu'il y a risque d'endormissement ou autre situation anormale.

Pour autoriser la transmission du paramètre de sortie d'intérêt vers un potentiel système externe, le terminal électronique 150 peut comprendre un étage de communication. Des protocoles connus de connexion (CAN, UART, USB) ou de transmission de données sans fil, (Wi-Fi, bluetooth, etc) peuvent par exemple être utilisés.

Afin de rendre autonome le dispositif 200, on pourra également prévoir une batterie, préférentiellement rechargeable, permettant d'alimenter en énergie le capteur de vibration 100 et/ou les différents étages précités du terminal électronique 150. Si le terminal 150 est déporté sur une zone du tableau de bord du véhicule, il pourra être alimenté par la batterie du véhicule.

Comme évoqué précédemment, le dispositif 200 peut se décliner sous différentes configurations :

- un dispositif portable et autonome, susceptible d'être positionné sur n'importe quel équipement de sécurité 1 de véhicule ;
- un dispositif fixe, dans lequel le terminal 150 est raccordé au capteur 100 en filaire ou intégré à un système externe fixe et plus complexe (système fixé sur le tableau de bord du véhicule ou intégré audit tableau de bord).

La présente invention concerne également un système de sécurité d'un véhicule comprenant un équipement de sécurité 1 solidaire (directement ou indirectement) du châssis du véhicule, en au moins un point de contact 2 (figure 1). L'équipement de sécurité 1 pourra être directement reliée au châssis, habituellement via au moins trois points de contact 2 par exemple pour une ceinture de sécurité. L'équipement de sécurité 1 pourra alternativement être indirectement relié au châssis, lorsque ledit équipement 1 est solidaire du siège du véhicule, lequel siège est solidaire du châssis, en un ou plusieurs points de contact 2.

Le système de sécurité comprend le dispositif 200 précité, pour la mesure d'au moins un signal vital périodique d'un individu (par exemple le conducteur du véhicule), fixé à l'équipement de sécurité 1 par une attache glissante 201.

Lorsqu'il est muni de l'organe d'atténuation mécanique 120, le dispositif 200 permet le recueil et l'analyse efficace d'un signal vital de l'individu dans le véhicule en fonctionnement car il isole le capteur de vibration 100 des vibrations mécaniques du moteur transmises à l'équipement de sécurité 1 par le châssis, comme cela sera illustré par la suite dans l'exemple d'application.

Un dispositif 200 selon l'invention, dépourvu de l'organe d'atténuation mécanique 120, peut également être mis en œuvre dans le système de sécurité. Dans ce cas, le système de sécurité comprend au moins un absorbeur d'énergie mécanique 210 placé en au moins un point de contact 2, de manière à isoler l'équipement de sécurité 1 des vibrations du châssis, en amont du capteur de vibration 100.

Dans le cas où l'équipement de sécurité 1 est relié au châssis en trois (ou éventuellement quatre) points de contact 2, il est avantageux de positionner un absorbeur d'énergie mécanique 210 en au moins un point de contact 2, voire en chacun des points de contact 2. Dans le cas où l'équipement de sécurité 1 est relié au siège, un absorbeur d'énergie mécanique 210 est préférentiellement positionné au(x) point(x) de contact 2 entre le siège et le châssis du véhicule.

Bien sûr, il est également possible de positionner un absorbeur d'énergie mécanique 2 au(x) point(s) de contact 2 entre le siège et le châssis, dans le cas où l'équipement de sécurité 1 est relié directement au châssis.

L'absorbeur d'énergie mécanique 210 va former un filtre mécanique et comprend donc un corps (masse) et un amortisseur (raideur, coefficient de frottement), comme cela a été décrit en référence à l'organe d'atténuation mécanique 120.

Enfin, il est envisageable de mettre en œuvre à la fois le dispositif 200 muni d'un organe d'atténuation mécanique 120 et les absorbeurs d'énergie mécanique 210 déportés sur tout ou partie des points de contact 2 directs ou indirects entre l'équipement de sécurité 1 et le châssis. Une telle configuration permet d'améliorer encore davantage la qualité du signal brut mesuré par le capteur de vibration 100, en limitant drastiquement les bruits et vibrations parasites liés au fonctionnement du moteur et au déplacement du véhicule.

Exemple de réalisation :

Un exemple de fabrication du capteur de vibration 100 et du dispositif 200 va maintenant être décrit. Bien sûr, cet exemple n'est pas limitatif car il existe d'autres procédés pour empiler et assembler différents types de couches, susceptibles d'être mis en œuvre pour réaliser le dispositif 200, conformément à l'invention.

Pour fabriquer l'empilement de couches 10 du capteur de vibration 100, il est notamment possible d'utiliser un procédé de transfert voisin de celui décrit par T.Dufay et al dans la publication « Flexible PZT thin film transferred on polymer substrate » (Surface and Coatings Technology, Elsevier, 2018, 343, pp.148-152).

Une solution de précurseur du PZT est déposée par centrifugation (« spin-coating ») sur un substrat sacrificiel (par exemple, en aluminium), pour former une couche visqueuse. Une ouverture est réalisée à travers ladite couche afin de permettre le passage d'une voie électrique. Puis, un traitement thermique à 650°C est appliqué pour cristalliser le PZT et former une couche active 11 en matériau piézoélectrique d'épaisseur 5 microns.

Une électrode de contact 12 en platine, d'épaisseur 400 nm, est déposée par une technique de dépôt chimique en phase vapeur (par exemple PECVD) sur la face supérieure (libre) de la couche active 11 en PZT, puis recouverte par une couche adhésive en polyuréthane. Une ouverture est également réalisée à travers l'empilement électrode / couche adhésive pour le passage de la voie électrique. Une couche temporaire en polymère (par exemple du PET), d'épaisseur 200 microns, est attachée à la couche adhésive en polyuréthane par thermocompression, pour faciliter la manipulation de la couche active 11. La couche temporaire est ouverte pour permettre le passage de la voie électrique, et remplie par de la colle conductrice, qui formera le via conducteur 14, en contact électrique avec l'électrode de contact 12. Le substrat sacrificiel est ensuite gravé chimiquement jusqu'à mettre à nu la face inférieure de la couche active 11 en PZT. L'autre électrode de contact 13 et le plot 12a, en contact électrique avec le via 14, sont formés par dépôt d'aluminium (environ 400 nm) sur ladite face inférieure du PZT.

Ce procédé de fabrication peut permettre l'élaboration d'un film de PZT présentant de grandes dimensions latérales, que l'on vient ensuite découper pour définir la couche active 11 aux dimensions latérales souhaitées pour son intégration dans le capteur de vibration 100 selon l'invention. Dans l'exemple décrit, la couche active 11 présente des dimensions latérales (selon le plan principal (x,y)) de 5 mm par 15 mm.

On choisit ensuite un circuit imprimé (PCB) 31 présentant une épaisseur de 100 microns, des dimensions latérales sensiblement identiques à celles de la couche active 11 et comportant deux bornes électriques 32,33. Un film conductif anisotrope (ACF) 20 est laminé sur le circuit imprimé 31. A l'aide d'une machine de manipulation (de type « Pick and Place »), la couche active 11 est positionnée en vis-à-vis de la couche de connexion 20, de sorte que chaque électrode 12a,13 (sur la face inférieure de la couche active 11) se trouve à l'aplomb d'une borne électrique 32,33 du circuit imprimé 31 ; puis un assemblage par thermocompression est opéré.

La couche temporaire en polymère peut alors être retirée.

Le circuit imprimé 31 est ensuite collé sur une membrane 35 en PVC, d'épaisseur 300 microns et de dimensions latérales (ou diamètre) 50 mm, pour finaliser la formation de la couche support 30. Une couche d'adaptation d'impédance 40 en silicone, d'épaisseur 3 mm, peut être assemblée par laminage, sérigraphie ou moulage contre la membrane 35. Une structure de rigidification 50 en polypropylène et un joint périphérique 60 en silicone sont fixés sur le pourtour de la membrane 35 par emboîtement.

Un capot en silicone, formant l'organe d'atténuation acoustique 110 au-dessus et à distance de la couche active 11, est moulé puis collé sur la structure de rigidification 50. Il présente une épaisseur de 2 mm.

Un organe d'atténuation mécanique 120 peut également être formé : il est composé de piliers en caoutchouc 120b, collés sur un corps 120a en acier d'épaisseur 5 mm. Le corps 120a est collé contre l'organe d'atténuation acoustique 110. Du côté de leur extrémité libre, les piliers 120b sont collés sur l'élément support 201a d'une attache 201, laquelle peut être associée à l'équipement de sécurité 1 d'un véhicule (une ceinture de sécurité 1 dans cet exemple). L'attache 201 peut par exemple être formée en polyoxyméthylène.

Pour le confort de l'utilisateur, l'ensemble peut être habillé d'un tissu 130 et/ou d'un mousse 140, en périphérie de la zone de mesure.

Dans cet exemple, le circuit imprimé 31 comprend un élément filaire 31b (nappe) qui permet de relier les bornes électriques 32,33 du circuit imprimé 31 au terminal électronique 150, via des prises de contact électrique. Le terminal 150 comprend les étages électroniques énoncés dans la description générale. Il est par exemple placé sous le siège de l'utilisateur.

Avec le dispositif 200 ainsi formé, un exemple d'application à la mesure du rythme cardiaque d'un conducteur est illustré sur les figures 7a et 7b. Pour la mesure du rythme cardiaque, le dispositif 200 est ajusté en hauteur le long de la ceinture de sécurité 1, de manière à être disposé sur le thorax de l'individu, sensiblement sur la gauche, la couche d'adaptation d'impédance 40 du capteur de vibration 100 étant placée en contact de ses vêtements, et l'organe d'atténuation mécanique 120 étant en contact avec la ceinture de sécurité 1, via l'attache glissante 201.

La figure 7a présente deux spectrogrammes A,B bruts, acquis sur une échelle de fréquences allant de 0 à 150 Hz, par un capteur de vibration 100 tel que précédemment décrit (fréquence d'acquisition 128 kHz). Dans le cas du spectrogramme A, le dispositif de mesure ne comporte ni l'organe d'atténuation acoustique 110, ni l'organe d'atténuation mécanique 120 ; le système de sécurité ne comporte pas non plus d'absorbeur d'énergie mécanique 210. Dans le cas du spectrogramme B, le dispositif 200 conforme à l'exemple précédemment décrit, comprend un organe d'atténuation acoustique 110 et un organe d'atténuation mécanique 120.

Lorsque le véhicule est à l'arrêt, les deux spectrogrammes A,B montrent des pics réguliers, qui, après traitement, procurent une information fiable sur le rythme cardiaque du conducteur ; cette information est fiable quel que soit le niveau sonore environnant dans le véhicule. Par contre, dès que le véhicule est en fonctionnement, les vibrations du moteur génèrent énormément de bruits et vibrations parasites, qui rendent le spectrogramme A inexploitable. Le dispositif 200 selon l'invention permet d'obtenir un spectrogramme B beaucoup moins bruité, grâce à la présence des organes d'atténuation acoustique 110 et mécanique 120. Notons qu'un résultat similaire pourrait être obtenu avec un dispositif 200 dépourvu de l'organe d'atténuation mécanique 120, dans le cas où le système de sécurité du véhicule comprend au moins un absorbeur d'énergie mécanique 210, au(x) point(s) de contact 2, direct ou indirect, entre la ceinture de sécurité 1 et le châssis.

On peut observer sur la figure 7b, un extrait B' d'environ 15s du spectrogramme B, dans la période au cours de laquelle le véhicule est en fonctionnement. On distingue plus nettement les pics réguliers représentatifs du rythme cardiaque du conducteur.

Le spectrogramme B" est obtenu en appliquant un filtre entre 40Hz et 70Hz et en normalisant le signal. Les pics pointés sur le spectrogramme B" peuvent être visualisés sous forme d'onde : c'est le signal B"', qui révèle les pics représentatifs du rythme cardiaque du conducteur. Ainsi, à partir du signal B"', il est possible d'extraire le signal périodique et/ou un paramètre de sortie, représentatif du rythme cardiaque de l'individu, avec un excellent niveau de précision.

Il est donc possible de détecter, de manière fiable, un changement du rythme cardiaque (ou de façon similaire, du rythme respiratoire) susceptible d'annoncer un endormissement du conducteur ou autre situation à risque. Dans un tel cas, le dispositif 200 est apte à déclencher une action (signal sonore ou lumineux par exemple) comme évoqué précédemment.

Comme cela vient d'être illustré et de manière générale, le dispositif 200 non intrusif de mesure d'un signal vital périodique selon la présente invention procure une information fiable quant au signal vital du conducteur, quel que soit l'environnement sonore dans le véhicule, moteur à l'arrêt ou en fonctionnement.

Bien entendu, l'invention n'est pas limitée aux modes de réalisation et aux exemples décrits, et on peut y apporter des variantes de réalisation sans sortir du cadre de l'invention tel que défini par les revendications.

**Revendications**

1. Dispositif (200) pour la mesure d'au moins un signal vital périodique d'un individu, destiné à être fixé à un équipement de sécurité (1) d'un véhicule de manière à être disposé entre l'individu et ledit équipement (1), le dispositif (200) comprenant :

   - un capteur de vibration (100) comportant :

      * un empilement de couches (10) s'étendant parallèlement à un plan principal (x,y) et incluant une couche active (11) en matériau piézoélectrique et deux électrodes de contact (12,13) disposées sur au moins une face de la couche active (11),
      * une couche support (30), flexible, destinée à se déformer à chaque pulsation du signal vital pour transmettre une déformation à la couche active (11) de l'empilement de couches (10), ladite couche support (30) s'étendant parallèlement au plan principal (x,y) et incluant un circuit imprimé (31) comportant deux bornes électriques (32,33), la couche support (30) étant destinée à être disposée contre l'individu,
      * une couche de connexion électrique (20), disposée entre l'empilement de couches (10) et la couche support (30), pour connecter chaque électrode de contact (12,13) à une borne électrique (32,33),

   - un organe d'atténuation acoustique (110), destiné à être disposé entre l'équipement de sécurité (1) et le capteur de vibration (100), ledit organe (110) étant solidaire de la couche support (30) et disposé au-dessus et à distance de l'empilement de couches (10).

2. Dispositif (200) selon la revendication 1, dans lequel l'organe d'atténuation acoustique comprend un capot, composé d'un matériau souple présentant une dureté comprise entre 10 Shore 00 et 80 Shore 00, et solidaire de la couche support (30) par sa périphérie.

3. Dispositif (200) selon la revendication 2, dans lequel le capot est hétérogène et comprend un deuxième matériau rigide choisi parmi les métaux ou les polymères présentant une dureté comprise entre 10 Shore D et 80 Shore D.

4. Dispositif (200) selon l'une des revendications précédentes, comprenant un organe d'atténuation mécanique (120), sur ou intégré en tout ou partie dans l'organe d'atténuation acoustique (110), ledit organe d'atténuation mécanique (120) étant destiné à être en contact, direct ou indirect, avec l'équipement de sécurité (1).

5. Dispositif (200) selon la revendication 4, dans lequel l'organe d'atténuation mécanique (120) comprend au moins un amortisseur et optionnellement un corps formant une masse.

6. Dispositif (200) selon l'une des revendications précédentes, dans lequel la couche active (11) de l'empilement de couches (10) présente une épaisseur inférieure ou égale à 20 microns et un module d'Young supérieur ou égal à 60 GPa.

7. Dispositif (200) selon l'une des revendications précédentes, comprenant une couche d'adaptation d'impédance (40), présentant une impédance acoustique comprise entre 5.10$^5$ Pa*s/m et 3.10$^6$ Pa*s/m, et disposée sur une face de la couche support (30) opposée à celle en contact avec la couche de connexion électrique (20).

8. Dispositif (200) selon la revendication précédente, dans lequel :

   - les électrodes de contact (12,12a,13) présentent une épaisseur cumulée inférieure à deux fois l'épaisseur de la couche active (11) ;
   - la couche support (30) est autoportée et présente une épaisseur inférieure ou égale à 500 microns ;
   - la couche d'adaptation d'impédance (40) présente une épaisseur supérieure ou égale à 10 microns.

9. Dispositif (200) selon l'une des revendications précédentes, dans lequel la couche support (30) inclut une membrane (35) disposée sur une face du circuit imprimé (31) opposée à celle en contact avec la couche de connexion électrique (20).

10. Dispositif (200) selon l'une des revendications précédentes, dans lequel la couche support (30) comprend une structure de rigidification (50), solidaire d'une zone périphérique de ladite couche support (30), l'organe d'atténuation acoustique (110) étant lui-même solidaire de la structure de rigidification (50).

11. Dispositif (200) selon l'une des revendications précédentes, comprenant un terminal électronique (150) raccordé au capteur de vibration (100), pour analyser et interpréter le signal brut et extraire le signal vital périodique ou un paramètre de sortie représentatif dudit signal vital périodique.

12. Dispositif (200) selon la revendication précédente, dans lequel le terminal électronique (150) comprend :

   - un étage analogique de conditionnement du signal brut mesuré par le capteur de vibration (100),
   - un étage de conversion analogique à digital du signal issu de l'étage de conditionnement,
   - un étage de traitement du signal digital, pour la mise en forme du signal digital et le calcul d'un paramètre de sortie représentatif dudit signal vital.

13. Système de sécurité d'un véhicule comprenant :

   - un équipement de sécurité (1), associé à un siège et solidaire d'un châssis du véhicule en au moins un point de contact (2) direct ou indirect,
   - un dispositif (200) pour la mesure d'au moins un signal vital périodique d'un individu, selon l'une des revendications précédentes, fixé à l'équipement de sécurité (1) par une attache glissante (201), et
   - au moins un absorbeur d'énergie mécanique (210) placé au niveau d'au moins un point de contact (2), de manière à isoler l'équipement de sécurité (1) des vibrations mécaniques du châssis.

14. Système de sécurité d'un véhicule selon la revendication précédente, dans lequel l'équipement de sécurité (1) est directement relié au châssis par au moins trois points de contact (2), et dans lequel un absorbeur d'énergie mécanique (210) est intégré à au moins un des points de contact (2).

15. Système de sécurité d'un véhicule selon la revendication 13, dans lequel l'équipement de sécurité (1) est relié au siège, lequel est solidaire du châssis par au moins un point de contact (2), et dans lequel un absorbeur d'énergie mécanique (210) est intégré audit point de contact (2).

**Patentansprüche**

1. Vorrichtung (200) zur Messung von mindestens einem periodischen Vitalsignal einer Person, wobei die dazu bestimmt ist, an einer Sicherheitsausrüstung (1) eines Fahrzeugs derart befestigt zu werden, dass sie zwischen der Person und der Ausrüstung (1) angeordnet ist, wobei die Vorrichtung (200) Folgendes umfasst:

   - einen Schwingungssensor (100), der Folgendes aufweist:

      * ein Schichtenstapel (10), der sich parallel zu einer Hauptebene (x, y) erstreckt und eine aktive Schicht (11) aus piezoelektrischem Material und zwei Kontaktelektroden (12, 13) enthält, die auf mindestens einer Seite der aktiven Schicht (11) angeordnet sind,
      * eine flexible Trägerschicht (30), die dazu bestimmt ist, sich bei jedem Impuls des Vitalsignals zu verformen, um eine Verformung an die aktive Schicht (11) des Schichtenstapels (10) zu übertragen, wobei sich die Trägerschicht (30) parallel zu der Hauptebene (x, y) erstreckt und eine Leiterplatte (31) enthält, die zwei elektrische Klemmen (32, 33) aufweist, wobei die Trägerschicht (30) dazu bestimmt ist, an der Person angeordnet zu werden,
* eine elektrische Verbindungsschicht (20), die zwischen dem Schichtenstapel (10) und der Trägerschicht (30) angeordnet ist, um jede Kontaktelektrode (12, 13) mit einer elektrischen Klemme (32, 33) zu verbinden,

- ein Schalldämpfungselement (110), das dazu bestimmt ist, zwischen der Sicherheitsausrüstung (1) und dem Schwingungssensor (100) angeordnet zu werden, wobei das Element (110) fest mit der Trägerschicht (30) verbunden ist und oberhalb und im Abstand von dem Schichtenstapel (10) angeordnet ist.

2. Vorrichtung (200) nach Anspruch 1, wobei das Schalldämpfungselement eine Abdeckung umfasst, die aus einem flexiblen Material mit einer Härte zwischen 10 Shore 00 und 80 Shore 00 besteht und an ihrem Umfang mit der Trägerschicht (30) verbunden ist.

3. Vorrichtung (200) nach Anspruch 2, wobei die Abdeckung heterogen ist und ein zweites starres Material umfasst, das aus Metallen oder Polymeren mit einer Härte zwischen 10 Shore D und 80 Shore D ausgewählt ist.

4. Vorrichtung (200) nach einem der vorhergehenden Ansprüche, die ein mechanisches Dämpfungselement (120) umfasst, das sich auf dem Schalldämpfungselement (110) befindet oder ganz oder teilweise in dieses integriert ist, wobei das mechanische Dämpfungselement (120) dazu bestimmt ist, direkt oder indirekt mit der Sicherheitsausrüstung (1) in Kontakt zu stehen.

5. Vorrichtung (200) nach Anspruch 4, wobei das mechanische Dämpfungselement (120) mindestens einen Dämpfer und optional einen Körper umfasst, der eine Masse bildet.

6. Vorrichtung (200) nach einem der vorhergehenden Ansprüche, wobei die aktive Schicht (11) des Schichtenstapels (10) eine Dicke von kleiner oder gleich 20 Mikrometern und einen Young-Modul von größer oder gleich 60 GPa aufweist.

7. Vorrichtung (200) nach einem der vorhergehenden Ansprüche, die eine Impedanzanpassungsschicht (40) umfasst, die eine akustische Impedanz zwischen $5,10^5$ Pa*s/m und $3,10^6$ Pa*s/m aufweist und auf einer der Trägerschicht (30) angeordnet ist, die derjenigen gegenüberliegt, die mit der elektrischen Verbindungsschicht (20) in Kontakt steht.

8. Vorrichtung (200) nach dem vorhergehenden Anspruch, wobei:

   - die Kontaktelektroden (12, 12a, 13) eine kumulative Dicke aufweisen, die kleiner ist als das Doppelte der Dicke der aktiven Schicht (11);
   - die Trägerschicht (30) selbsttragend ist und eine Dicke von kleiner oder gleich 500 Mikrometern aufweist;
   - die Impedanzanpassungsschicht (40) eine Dicke von größer oder gleich 10 Mikrometern aufweist.

9. Vorrichtung (200) nach einem der vorhergehenden Ansprüche, wobei die Trägerschicht (30) eine Membran (35) enthält, die auf einer Seite der Leiterplatte (31) angeordnet ist, die derjenigen gegenüberliegt, die mit der elektrischen Verbindungsschicht (20) in Kontakt steht.

10. Vorrichtung (200) nach einem der vorhergehenden Ansprüche, wobei die Trägerschicht (30) eine Versteifungsstruktur (50) umfasst, die fest mit einem Umfangsbereich der Trägerschicht (30) verbunden ist, wobei das Schalldämpfungselement (110) selbst fest mit der Versteifungsstruktur (50) verbunden ist.

11. Vorrichtung (200) nach einem der vorhergehenden Ansprüche, die ein elektronisches Endgerät (150) umfasst, das mit dem Schwingungssensor (100) verbunden ist, um das Rohsignal zu analysieren und zu interpretieren und das periodische Vitalsignal oder einen Ausgangsparameter, der repräsentativ für das periodische Vitalsignal ist, zu extrahieren.

12. Vorrichtung (200) nach dem vorhergehenden Anspruch, wobei das elektronische Endgerät (150) Folgendes umfasst:

   - eine analoge Stufe zur Konditionierung des von dem Schwingungssensor (100) gemessenen Rohsignals,
   - eine Stufe zur Analog-Digital-Umwandlung des aus der Konditionierungsstufe stammenden Signals,
   - eine Stufe zur Verarbeitung des digitalen Signals zur Bildung des digitalen Signals und zur Berechnung eines Ausgangsparameters, der repräsentativ für das Vitalsignal ist.

13. Sicherheitssystem eines Fahrzeugs, das Folgendes umfasst:

   - eine Sicherheitsausrüstung (1), die mit einem Sitz verbunden und an mindestens einem direkten oder indirekten Kontaktpunkt (2) fest mit einem Fahrgestell des Fahrzeugs verbunden ist,

- eine Vorrichtung (200) zur Messung von mindestens einem periodischen Vitalsignal einer Person nach einem der vorhergehenden Ansprüche, die mit einer Schiebehalterung (201) an der Sicherheitsausrüstung (1) befestigt ist, und
- mindestens einen mechanischen Energieabsorber (210), der an mindestens einem Kontaktpunkt (2) angeordnet ist, so dass die Sicherheitsausrüstung (1) von den mechanischen Schwingungen des Fahrgestells isoliert ist.

14. Sicherheitssystem eines Fahrzeugs nach dem vorhergehenden Anspruch, wobei die Sicherheitsausrüstung (1) durch mindestens drei Kontaktpunkte (2) direkt mit dem Fahrgestell verbunden ist, und wobei ein mechanischer Energieabsorber (210) in mindestens einen der Kontaktpunkte (2) integriert ist.

15. Sicherheitssystem eines Fahrzeugs nach Anspruch 13, wobei die Sicherheitsausrüstung (1) mit dem Sitz verbunden ist, der durch mindestens einen Kontaktpunkt (2) mit dem Fahrgestell fest verbunden ist, und wobei ein mechanischer Energieabsorber (210) in den Kontaktpunkt (2) integriert ist.


**Claims**

1. Device (200) for measuring at least one periodic vital signal of an individual, intended to be attached to safety equipment (1) of a vehicle so as to be disposed between the individual and said equipment (1), the device (200) comprising:

    - a vibration sensor (100) including:

        * a stack of layers (10) extending parallel to a main plane (x,y) and including an active layer (11) made of piezoelectric material and two contact electrodes (12, 13) disposed on at least one face of the active layer (11),
        * a flexible support layer (30) intended to deform with each pulse of the vital signal to transmit a deformation to the active layer (11) of the stack of layers (10), said support layer (30) extending parallel to the main plane (x,y) and including a printed circuit (31) comprising two electrical terminals (32.33), the support layer (30) being intended to be disposed against the individual,
        * an electrical connection layer (20), disposed between the stack of layers (10) and the support layer (30), to connect each contact electrode (12, 13) to an electrical terminal (32, 33),

    - an acoustic attenuation member (110), intended to be disposed between the safety equipment (1) and the vibration sensor (100), said member (110) being secured to the support layer (30) and disposed above and away from the stack of layers (10).

2. Device (200) according to claim 1, wherein the acoustic attenuation member comprises a cover, composed of a flexible material having a hardness between 10 Shore 00 and 80 Shore 00, and secured to the support layer (30) by its periphery.

3. Device (200) according to claim 2, wherein the cover is heterogeneous and comprises a second rigid material selected from metals or polymers having a hardness between 10 Shore D and 80 Shore D.

4. Device (200) according to one of the preceding claims, comprising a mechanical attenuation member (120), on or incorporated in whole or in part in the acoustic attenuation member (110), said mechanical attenuation member (120) being intended to be in direct or indirect contact with the safety equipment (1).

5. Device (200) according to claim 4, wherein the mechanical attenuation member (120) comprises at least one damper and optionally a body forming a mass.

6. Device (200) according to one of the preceding claims, wherein the active layer (11) of the stack of layers (10) has a thickness smaller than or equal to 20 microns and a Young's modulus higher than or equal to 60 GPa.

7. Device (200) according to one of the preceding claims, comprising an impedance matching layer (40), having an acoustic impedance comprised between $5.10^5$ Pa*s/m and $3 \cdot 10^6$ Pa*s/m, and disposed on a face of the support layer (30) opposite to that one in contact with the electrical connection layer (20).

8. Device (200) according to the preceding claim, wherein:

    - the contact electrodes (12, 12a, 13) have a total thickness smaller than twice the thickness of the active layer (11);
    - the support layer (30) is self-supporting and has a thickness less than or equal to 500 microns;
    - the impedance matching layer (40) has a thickness greater than or equal to 10 microns.

9. Device (200) according to one of the preceding claims, wherein the support layer (30) includes a membrane (35) disposed on a face of the printed circuit (31) opposite to the one in contact with the electrical connection layer (20).

10. Device (200) according to one of the preceding claims, wherein the support layer (30) comprises a stiffening structure (50), secured to a peripheral area of said support layer (30), the acoustic attenuation member (110) being itself secured to the stiffening structure (50).

11. Device (200) according to one of the preceding claims, comprising an electronic terminal (150) connected to the vibration sensor (100), for analysing and interpreting the raw signal and extracting the periodic vital signal or an output parameter representing said periodic vital signal.

12. Device (200) according to the preceding claim, wherein the electronic terminal (150) comprises:

- an analogue stage for conditioning the raw signal measured by the vibration sensor (100),
- a stage for analogue to digital conversion of the signal coming from the conditioning stage,
- a digital-signal processing stage, for shaping the digital signal and calculating an output parameter representing said vital signal.

13. Safety system of a vehicle comprising:

- safety equipment (1), associated with a seat and secured to a vehicle chassis at at least one point of direct or indirect contact (2),
- a device (200) for measuring at least one periodic vital sign of an individual, according to one of the preceding claims, secured to the safety equipment (1) by a sliding fastener (201), and
- at least one mechanical energy absorber (210) placed at at least one contact point (2), so as to isolate the safety equipment (1) from the mechanical vibrations of the chassis.

14. Safety system of a vehicle according to the preceding claim, wherein the safety equipment (1) is directly connected to the chassis by at least three contact points (2), and wherein a mechanical energy absorber (210) is incorporated in at least one of the contact points (2).

15. Safety system of a vehicle according to claim 13, wherein the safety equipment (1) is connected to the seat, which is secured to the chassis by at least one contact point (2), and wherein a mechanical energy absorber (210) is incorporated in said contact point (2).

[Fig. 1]

[Fig. 4]

(a)　　　　(b)　　　　(c)

[Fig. 2a]

[Fig. 2b]

[Fig. 3a]

[Fig. 3b]

[Fig. 5]

(a)          (b)          (c)

[Fig. 7a]

[Fig. 7b]

[Fig. 6a]

(i)

(ii)

(iii)

(iv)

[Fig. 6b]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 106725395

- CN 106500826 A

**Littérature non-brevet citée dans la description**

- Flexible PZT thin film transferred on polymer substrate. **T.DUFAY et al.** Surface and Coatings Technology. Elsevier, 2018, vol. 343, 148-152